# EUROPÄISCHE PATENTSCHRIFT

(11) **EP 3 440 181 B1**
(45) Veröffentlichungstag und Bekanntmachung des Hinweises auf die Patenterteilung: **12.02.2020**
(21) Anmeldenummer: 17713923.5
(22) Anmeldetag: 27.03.2017
(51) Int. Cl.: C11D 3/39, C07D 295/15

(54) **N-METHYLPIPERIDINGRUPPEN-HALTIGE ACYLHYDRAZONE**
N-METHYLPIPERIDINE GROUP-CONTAINING ACYL HYDRAZONES
ACYLHYDRAZONES POSSÉDANT DES GROUPES N-MÉTHYLPIPÉRIDINE

(30) Priorität: 04.04.2016 DE 102016205486
(43) Veröffentlichungstag der Anmeldung: 13.02.2019
(73) Patentinhaber: Henkel AG & Co. KGaA, 40589 Düsseldorf (DE)
(72) Erfinder: BLUHM, Nadine, 40233 Düsseldorf (DE); KROPF, Christian, 40724 Hilden (DE); SAUF, Silvia, 42555 Velbert (DE)
(86) Internationale Anmeldenummer: PCT/EP2017/057147
(87) Internationale Veröffentlichungsnummer: WO 2017/174382

(56) Entgegenhaltungen:
- WO-A1-2009/124855
- WO-A1-2012/080088
- DE-A1-102012 200 333
- DE-A1-102012 219 359
- DATABASE REGISTRY [Online] CHEMICAL ABSTRACTS SERVICE, COLUMBUS, OHIO, US; 16. November 1984 (1984-11-16), "piperidium, 1-methyl-1-[2-[2-[(5-nitro-2-furanyl)methy lene]hydrazinyl]-2-oxoethyl]- iodide (1:1)", XP002770789, gefunden im STN Database accession no. 1034-96-4
- DATABASE REGISTRY [Online] CHEMICAL ABSTRACTS SERVICE, COLUMBUS, OHIO, US; 23. Dezember 2004 (2004-12-23), "Piperidinium, 1-methyl-, 1[2-[2-[ (5-nitro-2-furanyl) methylene) hydrazinyl]-2- oxoethyl]-", XP002770790, gefunden im STN Database accession no. 802254-49-5

## Beschreibung

Die vorliegende Erfindung betrifft bestimmte Acylhydrazone und deren Anwendung zur Entfernung von Anschmutzungen von textilen oder harten Oberflächen, gegebenenfalls in Kombination mit persauerstoffhaltigem Bleichmittel, sowie Wasch- und Reinigungsmittel, welche derartige Acylhydrazone enthalten.

Herkömmliche Waschmittel mit Bleichmitteln auf Basis von Aktivsauerstoff, insbesondere in Gegenwart üblicher stöchiometrischer Aktivatoren (wie zum Beispiel TAED, NOBS, DECOBS, DOBA) zeigen eine gute Leistung bei Anwendungstemperaturen von 40°C und darüber. Bei niedrigeren Temperaturen wird ihre Leistung jedoch manchmal als verbesserungswürdig empfunden. So lassen sich bei Waschtemperaturen unter 40 °C nicht alle bleichbaren oder nicht bleichbaren Anschmutzungen immer ausreichend entfernen. Verbraucher tendieren dazu, bei immer niedrigeren Temperaturen zu waschen, erwarten aber trotzdem eine zufriedenstellende Leistung ihres Waschmittels. Hohe Waschtemperaturen gehen auch mit einem erhöhten Energieverbrauch einher, welcher aufgrund von Umweltschutz und Resourcenschonung zu vermeiden ist.

Aus der internationalen Patentanmeldung WO 2009/124855 sind Metallkomplexe mit Acylhydrazon-Liganden bekannt, die elektronenziehende Substituenten in der Nähe der Acylgruppe tragen. Die internationale Patentanmeldung WO 2012/080088 offenbart Acylhydrazone mit cyclischen Ammoniumgruppen als Substituenten in der Nähe der Acylgruppe.

Nun wurde gefunden, dass bestimmte N-Methylpiperidingruppen-haltigen Acylhydrazone insbesondere bei niedrigen Temperaturen eine besonders ausgeprägte bleichverstärkende Wirkung haben.

Die vorliegende Erfindung betrifft die Verwendung von Acylhydrazons der allgemeinen Formel I, wie definiert in den Ansprüchen 1 bis 4.

Solche Acylhydrazone verstärken die Bleichwirkung üblicher Bleichmittel auf Persauerstoffbasis. Weitere Gegenstände der Erfindung sind daher die Verwendung einer Kombination aus einem peroxidischen Bleichmittel mit einem derartigen Acylhydrazon zur Verbesserung der Schmutzentfernungsleistung von Wasch- oder Reinigungsmitteln bei deren Anwendung, die Verwendung eines derartigen Acylhydrazons zur Verbesserung der Bleichleistung von peroxidischem Bleichmittel in Wasch- oder Reinigungsmitteln bei deren Anwendung, und ein Verfahren zum Entfernen von Anschmutzungen von textilen oder harten Oberflächen durch In-Kontakt-Bringen der verschmutzen Oberfläche mit einer wässrigen Zubereitung, die peroxidisches Bleichmittel und derartiges Acylhydrazon enthält.

Es wurde beobachtet, dass die genannten Acylhydrazone auch in Abwesenheit peroxidischer Bleichmittel die Schmutzentfernungsleistung von Wasch- und Reinigungsmitteln insbesondere gegenüber bleichbaren oder enzymsensitiven Anschmutzungen verbessern. Weitere Gegenstände der Erfindung sind daher die Verwendung eines genannten Acylhydrazons zur Verbesserung der Schmutzentfernungsleistung von Wasch- oder Reinigungsmitteln bei deren Anwendung, und ein Verfahren zum Entfernen von Anschmutzungen von textilen oder harten Oberflächen durch In-Kontakt-Bringen der verschmutzen Oberfläche mit einer wässrigen Zubereitung, die ein derartiges Acylhydrazon enthält..

Ein weiterer Gegenstand der Erfindung ist ein Wasch- oder Reinigungsmittel, enthaltend ein genanntes Acylhydrazon und peroxidisches Bleichmittel.

Durch die erfindungsgemäße Verwendung des Acylhydrazons im erfindungsgemäßen Verfahren und bei Einsatz eines erfindungsgemäßen Mittels wird, was beim Verzicht auf die Anwesenheit von Bleichmitteln besonders bemerkenswert ist, insbesondere die Entfernung von bleichbaren Anschmutzungen verbessert. Ebenso wird die Entfernung von enzymsensitiven Anschmutzungen verbessert.

Unter bleichbaren Anschmutzungen sollen dabei solche verstanden werden, die gefärbt sind und nach dem Waschen in Gegenwart eines peroxidischen Bleichmittels, wie beispielsweise Natriumpercarbonat, und gegebenenfalls eines Bleichaktivators, wie beispielsweise N,N,N',N'-Tetraacetylethylendiamin, stärker entfernt werden oder zumindest einen helleren Farbton aufweisen als nach dem Waschen ohne das peroxidische Bleichmittel und gegebenenfalls den Bleichaktivator. Die bleichbaren Anschmutzungen enthalten üblicherweise polymerisierbare Substanzen, insbesondere Farbstoffe, wobei es sich bei den Farbstoffen vorzugsweise um polyphenolische Farbstoffe, insbesondere um Flavonoide, vor allem um Anthocyanidine oder Anthocyane oder Oligomere dieser Verbindungen handelt. Neben der Entfernung von Anschmutzungen in den Farben grün, gelb, rot oder blau kommt auch die von Anschmutzungen in Zwischenfarben, insbesondere violett, lila, braun, purpurfarben oder rosa, und auch von Anschmutzungen in Betracht, die eine grüne, gelbe, rote, violette, lilafarbene, braune, purpurfarbene, rosafarbene oder blaue Tönung aufweisen, ohne im Wesentlichen selbst komplett aus dieser Farbe zu bestehen. Die genannten Farben können insbesondere auch jeweils hell oder dunkel sein. Es handelt sich hierbei vorzugsweise um Anschmutzungen, insbesondere um Flecken von Gras, Früchten oder Gemüse, insbesondere auch um Anschmutzungen durch Lebensmittelprodukte, wie beispielsweise Gewürze, Saucen, Chutneys, Currys, Pürees und Marmeladen, oder Getränke, wie beispielsweise Kaffee, Tee, Weine und Säfte, die entsprechende grüne, gelbe, rote, violette, lilafarbene, braune, purpurfarbene, rosafarbene und/oder blaue Farbstoffe enthalten.

Unter enzymsensitiven Anschmutzungen sollen dabei solche verstanden werden, die nach dem Waschen in Gegenwart von Enzymen stärker entfernt werden oder, wenn sie gefärbt sind, einen helleren Farbton aufweisen als nach dem Waschen ohne das Enzym. Vorzugsweise handelt es sich um proteinhaltige, polysaccharidhaltige und/oder fetthaltige Anschmutzungen, wie beispielsweise Ei, Blut, Stärke, Mannan, Gras, oder Soßen.

Die Acylhydrazone der allgemeinen Formel (I) können in E- oder Z-Konfiguration vorliegen; gegebenenfalls können sie in einer ihrer tautomeren Formen oder als Mischung aus diesen vorliegen.

Das Anion A⁻ in den Acylhydrazonen der allgemeinen Formel (I) wird vorzugsweise aus der Gruppe umfassend Carboxylat wie Acetat, Lactat, Citrat, Tartrat oder Succinat, Perchlorat, Tetrafluoroborat, Hexafluorophosphat, Alkylsulfonat, Alkylsulfat, Hydrogensulfat, Sulfat, Dihydrogenphosphat, Hydrogenphosphat, Phosphat, Isocyanat, Rhodanid, Nitrat, Fluorid, Chlorid, Bromid, Hydrogencarbonat und Carbonat sowie deren Mischungen ausgewählt, wobei bei mehrwertigen Anionen der Ladungsausgleich durch entsprechend mehrere kationische Acylhydrazone oder gegebenenfalls durch die Anwesenheit zusätzlicher Kationen wie Natrium- oder Ammoniumionen erreicht werden kann. Bevorzugte Alkylgruppen in den Alkylsulfonaten und Alkylsulfaten sind solche mit 1 bis 18 C-Atomen, die verzweigt oder vorzugsweise linear sind.

In der allgemeinen Formel (I), steht R¹ für einen Rest 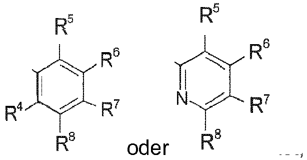 , wobei R⁵=OH und R² = R⁴ = R⁶ = R⁷ = R⁸ = H.

Besonders bevorzugt ist das Acylhydrazon der Formel

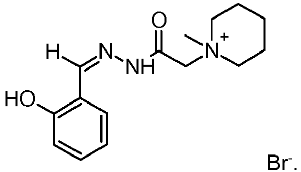

Acylhydrazone der allgemeinen Formel (I) können in im Prinzip bekannter Weise durch Umsetzung von N-Methylpiperidin mit Halogenessigsäureestern, anschließende Hydrazinolyse der Esterfunktion und Umsetzung mit Aldehyden hergestellt werden.

Die Mittel, in denen erfindungsgemäße Acylhydrazone eingesetzt werden, können Reinigungsmittel für harte Oberflächen wie beispielsweise von Geschirr, Fußboden- oder Wandfliesen, Toiletten, Arbeitsoberflächen, oder Waschmittel für beispielsweise Textilien, Teppiche oder Naturfasern sein. Zu den Waschmitteln zählen ferner Waschhilfsmittel, die bei der manuellen oder maschinellen Textilwäsche zum eigentlichen Waschmittel zudosiert werden, um eine weitere, vom eigentlichen Waschmittel nicht oder nur in unzureichendem Maß bereitgestellte Wirkung zu erzielen. Ferner zählen zu Waschmitteln im Rahmen der Erfindung auch Waschvor- und -nachbehandlungsmittel, also solche Mittel, mit denen das Wäschestück vor oder nach der eigentlichen Wäsche in Kontakt gebracht wird, beispielsweise zum Anlösen hartnäckiger Verschmutzungen oder zur Erzielung von weichem Griff und angenehmem Trageverhalten.

Die Mittel können teilchenförmig oder flüssig sein. "Flüssig" bedeutet dabei, dass das Wasch- oder Reinigungsmittel bei Raumtemperatur, das heißt bei etwa 20 °C, in flüssiger Form und insbesondere fließfähig vorliegt und damit beispielsweise aus einem Behälter ausgeschüttet werden kann.

In festen und insbesondere in flüssigen Mitteln der Erfindung oder solchen, die im Rahmen der erfindungsgemäßen Verwendung oder des erfindungsgemäßen Verfahrens eingesetzt werden, kann das Acylhydrazon und/oder das peroxidische Bleichmittel in gelöster oder suspendierter Form vorliegen; in der letztgenannten Ausführungsform können sie oder zumindest eines von ihnen in Form von Pulvern oder gewünschtenfalls übliche inerte Trägermaterialien enthaltenden Granulaten vorliegen, die auch in im Prinzip bekannter Weise umhüllt sein können.

Der Einsatz erfindungswesentlicher Acylhydrazone führt zur deutlich verbesserten Entfernung von bleichbaren und nicht-bleichbaren Anschmutzungen im Waschprozess bei niedrigen Temperaturen. Außerdem ist ein "Finetuning" der Fleckentfernungsleistung durch Optimierung des Bleichsystems und eine Energieersparnis durch die verbesserte Fleckentfernungsleistung und dadurch bedingte niedrigere Waschtemperaturen sowie eine Wasserersparnis durch Vermeidung von multiplen Waschvorgängen, da Flecken auf Anhieb beim ersten Waschgang entfernt werden, gegeben.

Im Rahmen der erfindungsgemäßen Verwendung und des erfindungsgemäßen Verfahrens ist bevorzugt, wenn die Konzentration des Acylhydrazons in der insbesondere wässrigen Wasch- beziehungsweise Reinigungsflotte, wie sie beispielsweise bei der Handwäsche, in Waschmaschinen, aber auch bei der Reinigung von Teppichen oder Polstermaterialien oder beim Reinigen harter Oberflächen, wie Kacheln, Fliesen oder Geschirr, das auch unter Einsatz von üblichen Geschirrspülmaschinen erfolgen kann, zum Einsatz kommt, 0,5 µmol/l bis 500 µmol/l, insbesondere 5 µmol/l bis 100 µmol/l beträgt. Vorzugsweise liegt die Konzentration an Mangan-, Titan-, Cobalt-, Nickel- oder Kupferionen in der wässrigen Wasch- beziehungsweise Reinigungsflotte im Bereich von 0,1 µmol/l bis 500 µmol/l, insbesondere 1 µmol/l bis 100 µmol/l. Bevorzugte Persauerstoffkonzentrationen (berechnet als H₂O₂) in der Wasch- beziehungsweise Reinigungsflotte liegen im Bereich von 0,001 g/l bis 10 g/l, insbesondere von 0,1 g/l bis 1 g/l und besonders bevorzugt von 0,2 g/l bis 0,5 g/l. Die erfindungsgemäße Verwendung wird vorzugsweise bei Temperaturen im Bereich von 10 °C bis 95 °C, insbesondere von 20 °C bis unter 40 °C durchgeführt. Die Wasserhärte des zur Zubereitung der wässrigen Wasch- beziehungsweise Reinigungsflotte zum Einsatz kommenden Wassers liegt vorzugsweise im Bereich von 0°dH bis 27°dH, insbesondere 0°dH bis 21°dH. In der Waschflotte liegt die Wasserhärte vorzugsweise im Bereich von 0°dH bis 16°dH, insbesondere 0°dH bis 3°dH, was beispielsweise durch den Einsatz üblicher Buildermaterialien oder Wasserenthärter erreicht werden kann, falls das zur Herstellung der Waschflotte eingesetzte Wasser nicht entsprechend weich ist. Die erfindungsgemäße Verwendung und das erfindungsgemäße Verfahren wird vorzugsweise bei pH-Werten im Bereich von pH 5 bis pH 12, insbesondere von pH 7 bis pH 11 durchgeführt.

Die erfindungsgemäßen Verwendungen und die erfindungsgemäßen Verfahren können besonders einfach durch den Einsatz eines erfindungsgemäßen Wasch- oder Reinigungsmittels, das ein genanntes Acylhydrazon oder gewünschtenfalls einen durch Komplexbildung mit einem genannten Übergangsmetallion aus diesem zugänglichen Metallkomplex enthält, realisiert werden.

Wasch- und Reinigungsmittel, die als insbesondere pulverförmige Feststoffe, in nachverdichteter Teilchenform, als homogene Lösungen oder Suspensionen vorliegen können, und die erfindungswesentliches Acylhydrazon enthalten oder im Rahmen der erfindungsgemäßen Verwendungen oder Verfahren eingesetzt werden, können im Prinzip alle bekannten und in derartigen Mitteln üblichen Inhaltsstoffe enthalten. Die Mittel können insbesondere Buildersubstanzen, Tenside, Wasser, wassermischbare organische Lösungsmittel, Bleichmittel, Enzyme, Sequestrierungsmittel, Elektrolyte, pH-Regulatoren, Polymere mit Spezialeffekten, wie soil release-Polymere, Farbübertragungsinhibitoren, Vergrauungsinhibitoren, knitterreduzierende polymere Wirkstoffe und formerhaltende polymere Wirkstoffe, und weitere Hilfsstoffe, wie optische Aufheller, Schaumregulatoren, Farb- und Duftstoffe, enthalten.

Vorzugsweise sind in Wasch- oder Reinigungsmitteln 0,001 Gew.-% bis 5 Gew.-%, insbesondere 0,05 Gew.-% bis 2 Gew.-% des genannten Acylhydrazons enthalten. Die Angabe von "Gew.-%" bezieht sich hier und im Folgenden auf das Gesamtgewicht des Mittels, sofern nicht anders angegeben. Bevorzugt ist auch, dass das Mittel zusätzlich ein Mangan-, Titan-, Cobalt-, Nickel- oder Kupfer-Salz und/oder einen Mangan-, Titan-, Cobalt-, Nickel- oder Kupfer-Komplex ohne einen Liganden, welcher einem genannten Acylhydrazon entspricht, enthält. Dann liegt das Molverhältnis des genannten Übergangsmetalls oder der Summe der genannten Übergangsmetalle zu Acylhydrazon vorzugsweise im Bereich von 0,001:1 bis 2:1, insbesondere 0,01:1 bis 1:1. In einer weiteren bevorzugten Ausgestaltung erfindungsgemäßer Mittel sind in diesen 0,05 Gew.-% bis 1 Gew.-%, insbesondere 0,1 Gew.-% bis 0,5 Gew.-% an bleichkatalysierendem Komplex, der ein genanntes Acylhydrazon als Liganden aufweist, enthalten. Bevorzugtes Übergangsmetall ist Mn.

Als in den Mitteln gegebenenfalls enthaltene Persauerstoffverbindungen (peroxidische Bleichmittel) kommen insbesondere organische Persäuren beziehungsweise persaure Salze organischer Säuren, Wasserstoffperoxid und unter den Waschbedingungen in Wasser Wasserstoffperoxid abgebende anorganische oder organische Verbindungen, in Betracht. Wasserstoffperoxid kann dabei auch mit Hilfe eines enzymatischen Systems, das heißt einer Oxidase und ihres Substrats, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat, Alkaliperborat-Tetrahydrat oder Wasserstoffperoxid in Form wässriger Lösungen, die 3 Gew.-% bis 10 Gew.-% Wasserstoffperoxid enthalten, eingesetzt. Vorzugsweise sind Persauerstoffverbindungen in Mengen von bis zu 50 Gew.-%, insbesondere von 5 Gew.-% bis 30 Gew.-%, in Wasch- oder Reinigungsmitteln vorhanden. Vorzugsweise werden als peroxidische Bleichmittel H₂O₂ oder in Wasser H₂O₂ freisetzende Substanzen eingesetzt, zu denen insbesondere Alkaliperborate, Alkalipercarbonate und Harnstoffperhydrat gehören; möglich ist jedoch auch der Einsatz von Peroxocarbonsäuren wie Diperoxodecandicarbonsäure oder Phthalimidopercapronsäure, anderen Säuren oder sauren Salzen, wie Alkalipersulfaten oder -peroxodisulfaten oder Caroaten, oder Diacylperoxiden oder Tetraacyldiperoxiden.

Die Leistung der genannten Acylhydrazone kann gegebenenfalls durch die Anwesenheit von Mangan-, Titan-, Cobalt-, Nickel- oder Kupferionen, vorzugsweise Mn(II)-, Mn(III)-, Mn(IV)- und/oder Mn(V)-, Cu(I)-, Cu(II)- und/oder Cu(III)-, Fe(I)-, Fe(II)-, Fe(III)- und/oder Fe(IV), Co(I)-, Co(II)- und/oder Co(III)-, Ni(I)-, Ni(II)- und/oder Ni(III)-, Ti(II)-, Ti(III)- und/oder Ti(IV)-Ionen, und besonders bevorzugt ausgewählt aus Mn(II)-, Mn(III)-, Mn(IV)-, Mn(V)-, Cu(I)-, Cu(II)-, Cu(III)-, Fe(I)-, Fe(II)-, Fe(III)-, Fe(IV)-, Co(I)-, Co(II)- oder Co(III)-Ionen oder Mischungen aus diesen, weiter verstärkt werden; gewünschtenfalls können auch Komplexverbindungen der genannten Metallzentralatome mit mindestens einem der genannten Acylhydrazone als Liganden eingesetzt werden. Ein Komplex, der einen Acylhydrazonliganden aufweist, kann den entsprechenden Liganden einmal oder auch mehrfach, insbesondere zweimal, aufweisen. Er kann ein- oder gegebenenfalls zwei- oder mehrkernig sein. Er kann außerdem weitere Neutral-, Anion- oder Kationliganden, wie beispielsweise H₂O, NH₃, CH₃OH, Acetylaceton, Terpyridin, organische Anionen, wie beispielsweise Citrat, Oxalat, Tartrat, Formiat, ein C₂₋₁₈-Carboxylat, ein C₁₋₁₈-Alkylsulfat, insbesondere Methosulfat, oder ein entsprechendes Alkansulfonat, anorganische Anionen, wie beispielsweise Halogenid, insbesondere Chlorid, Perchlorat, Tetrafluoroborat, Hexafluorophosphat, Nitrat, Hydrogensulfat, Hydroxid oder Hydroperoxid. Er kann auch verbrückende Liganden, wie beispielsweise Alkylendiamine, aufweisen.

In weiteren bevorzugten Ausgestaltungen der Erfindung wird in Gegenwart von H₂O₂ freisetzenden Persauerstoffverbindungen eine unter Perhydrolysebedingungen eine Peroxocarbonsäure oder Peroxoimidsäure ausbildende Verbindung und/oder eine übliche die Bleiche aktivierende Übergangsmetallkomplexverbindung zusammen mit dem Acylhydrazon eingesetzt. Verbindungen, die unter Perhydrolysebedingungen gegebenenfalls substituierte Perbenzoesäure und/oder Peroxocarbonsäuren mit 1 bis 10 C-Atomen, insbesondere 2 bis 4 C-Atomen ergeben, sind dabei bevorzugt. Geeignet sind übliche Bleichaktivatoren, die O- und/oder N-Acylgruppen tragen, zum Beispiel mehrfach acylierte Alkylendiamine, insbesondere Tetraacetylethylendiamin (TAED), acylierte Glykolurile, insbesondere Tetraacetylglykoluril (TAGU), acylierte Triazinderivate, insbesondere 1,5-Diacetyl-2,4-dioxohexahydro-1,3,5-triazin (DADHT), acylierte Phenylsulfonate und -carboxylate, insbesondere Nonanoyloxy- oder Isononanoyloxy-benzolsulfonat oder -benzoat, acylierte mehrwertige Alkohole, insbesondere Triacetin, Ethylenglykoldiacetat und 2,5-Diacetoxy-2,5-dihydrofuran sowie acetyliertes Sorbit und Mannit, und acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, sowie kationische Nitrilderivate wie Trimethylammoniumacetonitril-Salze. Vorzugsweise werden unter Perhydrolysebedingungen Peroxocarbonsäure oder Peroxoimidsäure ausbildende Verbindung und Acylhydrazon in Molverhältnissen im Bereich von 4:1 bis 100:1, insbesondere von 25:1 bis 50:1 eingesetzt, wenn solche zusätzlich zu dem Acylhydrazon vorhanden sind. Diese fakultativen Verbindungen sind in Wasch- und Reinigungsmitteln insbesondere in Mengen von 0,01 Gew.-% bis 10 Gew.-% und besonders bevorzugt von 1 Gew.-% bis 3 Gew.-%, vorhanden. Die Bleichaktivatoren können zur Vermeidung der Wechselwirkung mit den Bleichmittelverbindungen bei der Lagerung in bekannter Weise mit Hüllsubstanzen überzogen beziehungsweise granuliert worden sein, wobei mit Hilfe von Carboxymethylcellulose granuliertes Tetraacetylethylendiamin mit mittleren Korngrößen von 0,01 mm bis 0,8 mm, granuliertes 1,5-Diacetyl-2,4-dioxohexahydro-1,3,5-triazin, und/oder in Teilchenform konfektioniertes Trialkylammoniumacetonitril besonders bevorzugt ist.

Ein Mittel kann zur Verstärkung der Desinfektionswirkung, beispielsweise gegenüber speziellen Keimen, zusätzlich zu den bisher genannten Inhaltsstoffen übliche antimikrobielle Wirkstoffe enthalten. Derartige antimikrobielle Zusatzstoffe sind in Desinfektionsmitteln vorzugsweise in Mengen bis zu 10 Gew.-%, insbesondere von 0,1 Gew.-% bis 5 Gew.-%, enthalten, können aber auch ganz fehlen, da beim Einsatz der erfindungswesentlichen Acylhydrazone eine signifikante Abnahme unerwünschter Mikroorganismen zu beobachten ist.

Die Mittel können ein oder mehrere Tenside enthalten, wobei insbesondere anionische Tenside, nichtionische Tenside und deren Gemische in Frage kommen, aber auch kationische und/oder amphotere Tenside enthalten sein können. Geeignete nichtionische Tenside sind insbesondere Alkylglykoside und Alkoxylierungsprodukte von Alkylglykosiden oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Alkoxylierungsprodukte von N-Alkyl-aminen, vicinalen Diolen, Fettsäureestern und Fettsäureamiden, die hinsichtlich des Alkylteils den genannten Alkoholderivaten entsprechen, sowie von Alkylphenolen mit 5 bis 12 C-Atomen im Alkylrest brauchbar.

Als nichtionische Tenside werden insbesondere alkoxylierte, vorzugsweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann oder lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Nichtionisches Tensid vom Typ der alkoxylierten Alkohole ist in Wasch- oder Reinigungsmitteln vorzugsweise in Mengen von 0,5 Gew.-% bis 10 Gew.-%, insbesondere von 4 Gew.-% bis 6 Gew.-% enthalten.

Unter den anionischen Tensiden sind insbesondere Seifen und solche, die Sulfat- oder SulfonatGruppen mit bevorzugt Alkaliionen als Kationen enthalten, zu nennen. Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den Tensiden des Sulfat-Typs gehören die Salze der Schwefelsäurehalbester von Fettalkoholen mit 12 bis 18 C-Atomen. Zu den Tensiden vom Sulfonat-Typ gehören Alkansulfonate mit 12 bis 18 C-Atomen, Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Anionische Tenside vom Typ der linearen Alkylbenzolsulfonate sind die Salze, vorzugsweise die Alkalisalze und insbesondere die Natriumsalze von mit linearen Alkylgruppen mit 6 bis 19, vorzugsweise 7 bis 15 und insbesondere 9 bis 13 C-Atomen substituierter Benzolsulfonsäuren. Ein ganz besonders bevorzugter Vertreter ist Natriumdodecylbenzolsulfonat. Anionisches Tensid vom Typ der linearen Alkylbenzolsulfonate ist in Wasch- oder Reinigungsmitteln vorzugsweise in Mengen von 0,5 Gew.-% bis 10 Gew.-%, insbesondere von 4 Gew.-% bis 6 Gew.-% enthalten.

Anionische Tenside vom Typ der Ethersulfate sind die Salze der Schwefelsäuremonoester von mit Alkylenoxid umgesetzten geradkettigen oder verzweigten C₇₋₂₁-Alkoholen, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO. Geeignete Ethersulfate sind beispielsweise Verbindungen der Formel R²¹-O-(AO)ₙ-SO₃⁻ X⁺, in der R²¹ für einen linearen oder verzweigten, substituierten oder unsubstituierten Alkylrest, vorzugsweise für einen linearen, unsubstituierten Alkylrest, besonders bevorzugt für einen Fettalkoholrest, steht. Bevorzugte Reste R²¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R²¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen. AO steht für eine Ethylenoxid- (EO) oder Propylenoxid- (PO) Gruppierung, vorzugsweise für eine Ethylenoxidgruppierung. Der Index n steht für eine Zahl im Bereich von 1 bis 50, vorzugsweise von 1 bis 20 und insbesondere von 2 bis 10; ganz besonders bevorzugt steht n für Zahlen im Bereich von 2 bis 8. Die angegebenen Alkoxylierungsgrade n stellen in der Regel statistische Mittelwerte dar, die eine ganze oder eine gebrochene Zahl sein können. X steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH₄⁺, ½ Zn²⁺,½ Mg²⁺,½ Ca²⁺,½ Mn²⁺, und deren Mischungen. Anionisches Tensid vom Typ der Ethersulfate ist in Wasch- oder Reinigungsmitteln vorzugsweise in Mengen von 1 Gew.-% bis 10 Gew.-%, insbesondere von 5 Gew.-% bis 8 Gew.-% enthalten.

Die Mittel können, insbesondere wenn es sich bei ihnen um solche handelt, die für die Behandlung von Textilien vorgesehen sind, als kationische Oberflächenaktivsubstanzen mit textilweichmachender Wirkung insbesondere einen oder mehrere der kationischen, textilweichmachenden Stoffe der allgemeinen Formeln X, XI oder XII enthalten: 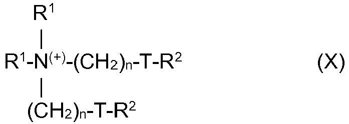 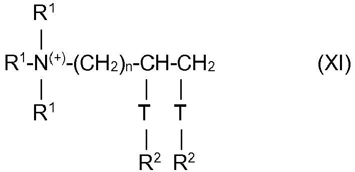 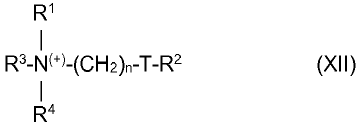 worin jede Gruppe R¹ unabhängig voneinander ausgewählt ist aus C₁₋₆-Alkyl-, -Alkenyl- oder -Hydroxyalkylgruppen; jede Gruppe R² unabhängig voneinander ausgewählt ist aus C₈₋₂₈-Alkyl- oder -Alkenylgruppen; R³ = R¹ oder (CH₂)ₙ-T-R²; R⁴ = R¹ oder R² oder (CH₂)ₙ-T-R²; T = -CH₂-, -O-CO- oder -CO-O- und n eine ganze Zahl von 0 bis 5 ist. Die kationischen Tenside weisen übliche Anionen in zum Ladungsausgleich notwendiger Art und Anzahl auf, wobei diese neben beispielsweise Halogeniden auch aus den anionischen Tensiden ausgewählt werden können. In bevorzugten Ausführungsformen kommen als kationische Tenside Hydroxyalkyl-trialkyl-ammoniumverbindungen, insbesondere C₁₂₋₁₈-Alkyl(hydroxyethyl)dimethylammoniumverbindungen, und vorzugsweise deren Halogenide, insbesondere Chloride, zum Einsatz. Textibehandlungsmittel enthalten bis zu 25 Gew.-%, insbesondere 0,5 Gew.-% bis 15 Gew.-% kationisches Tensid.

Ein Wasch- oder Reinigungsmittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylen-phosphonsäure) und 1-Hydroxyethan-1,1-diphosphonsäure, polymere Hydroxyverbindungen wie Dextrin sowie polymere (Poly-)carbonsäuren, insbesondere durch Oxidation von Polysacchariden beziehungsweise Dextrinen zugänglichen Polycarboxylate, und/oder polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättigter Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/oder einem veresterten Vinylalkohol oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure, wobei Maleinsäure besonders bevorzugt ist, und/oder ein Derivat einer Allylsulfonsäure, die in 2-Stellung mit einem Alkyl- oder Arylrest substituiert ist, sein. Derartige Polymere weisen im Allgemeinen eine relative Molekülmasse zwischen 1 000 und 200 000 auf. Weitere bevorzugte Copolymere sind solche, die als Monomere Acrolein und Acrylsäure/Acrylsäuresalze beziehungsweise Vinylacetat aufweisen. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen Mitteln eingesetzt.

Als wasserlösliche anorganische Buildermaterialien kommen insbesondere polymere Alkaliphosphate, die in Form ihrer alkalischen neutralen oder sauren Natrium- oder Kaliumsalze vorliegen können, in Betracht. Beispiele hierfür sind Tetranatriumdiphosphat, Dinatriumdihydrogendiphosphat, Pentanatriumtriphosphat, sogenanntes Natriumhexametaphosphat sowie die entsprechenden Kaliumsalze beziehungsweise Gemische aus Natrium- und Kaliumsalzen. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das nach den Angaben der deutschen Patentschrift DE 24 12 837 bestimmt werden kann, liegt in der Regel im Bereich von 100 mg bis 200 mg CaO pro Gramm.

Geeignete Substitute beziehungsweise Teilsubstitute für das genannte Alumosilikat sind kristalline Alkalisilikate, die allein oder im Gemisch mit amorphen Silikaten vorliegen können. Die als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisitikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können eingesetzt werden. In einer weiteren bevorzugten Ausführungsform wird ein kristallines Natriumschichtsilikat mit einem Modul von 2 bis 3 eingesetzt, wie es n aus Sand und Soda hergestellt werden kann. Kristalline Natriumsilikate mit einem Modul im Bereich von 1,9 bis 3,5 werden in einer weiteren bevorzugten Ausführungsform eingesetzt. In einer bevorzugten Ausgestaltung setzt man ein granulares Compound aus Alkalisilikat und Alkalicarbonat ein, wie es zum Beispiel unter dem Namen Nabion® 15 im Handel erhältlich ist. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind in Wasch- oder Reinigungsmitteln vorzugsweise in Mengen bis zu 60 Gew.-%, insbesondere von 5 Gew.-% bis 40 Gew.-%, enthalten.

In einer bevorzugten Ausgestaltung weist das Mittel einen wasserlöslichen Builderblock auf. Durch die Verwendung des Begriffes "Builderblock" soll hierbei ausgedrückt werden, dass die Mittel keine weiteren Buildersubstanzen enthalten als solche, die wasserlöslich sind, das heißt sämtliche in dem Mittel enthaltenen Buildersubstanzen sind in dem so charakterisierten "Block" zusammengefasst, wobei allenfalls die Mengen an Stoffen ausgenommen sind, die als Verunreinigungen beziehungsweise stabilisierende Zusätze in geringen Mengen in den übrigen Inhaltsstoffen der Mittel handelsüblicherweise enthalten sein können. Unter dem Begriff "wasserlöslich" soll dabei verstanden werden, dass sich der Builderblock bei der Konzentration, die sich durch die Einsatzmenge des ihn enthaltenden Mittels bei den üblichen Bedingungen ergibt, rückstandsfrei löst. Vorzugsweise sind mindestens 15 Gew.-% und bis zu 55 Gew.-%, insbesondere 25 Gew.-% bis 50 Gew.-% an wasserlöslichem Builderblock in den Mitteln enthalten. Dieser setzt sich vorzugsweise zusammen aus den Komponenten
a) 5 Gew.-% bis 35 Gew.-% Citronensäure, Alkalicitrat und/oder Alkalicarbonat, welches auch zumindest anteilig durch Alkalihydrogencarbonat ersetzt sein kann,
b) bis zu 10 Gew.-% Alkalisilikat mit einem Modul im Bereich von 1,8 bis 2,5,
c) bis zu 2 Gew.-% Phosphonsäure und/oder Alkaliphosphonat,
d) bis zu 50 Gew.-% Alkaliphosphat, und
e) bis zu 10 Gew.-% polymerem Polycarboxylat,
wobei die Mengenangaben sich auf das gesamte Wasch- beziehungsweise Reinigungsmittel beziehen. Dies gilt auch für alle folgenden Mengenangaben, sofern nicht ausdrücklich anders angegeben.

In einer bevorzugten Ausführungsform enthält der wasserlösliche Builderblock mindestens 2 der Komponenten b), c), d) und e) in Mengen größer 0 Gew.-%.

Hinsichtlich der Komponente a) sind in einer bevorzugten Ausführungsform 15 Gew.-% bis 25 Gew.-% Alkalicarbonat, welches zumindest anteilig durch Alkalihydrogencarbonat ersetzt sein kann, und bis zu 5 Gew.-%, insbesondere 0,5 Gew.-% bis 2,5 Gew.-% Citronensäure und/oder Alkalicitrat enthalten. In einer alternativen Ausführungsform sind als Komponente a) 5 Gew.-% bis 25 Gew.-%, insbesondere 5 Gew.-% bis 15 Gew.-% Citronensäure und/oder Alkalicitrat und bis zu 5 Gew.-% , insbesondere 1 Gew.-% bis 5 Gew.-% Alkalicarbonat, welches zumindest anteilig durch Alkalihydrogencarbonat ersetzt sein kann, enthalten. Falls sowohl Alkalicarbonat wie auch Alkalihydrogencarbonat vorhanden sind, weist die Komponente a) Alkalicarbonat und Alkalihydrogencarbonat vorzugsweise im Gewichtsverhältnis von 10:1 bis 1:1 auf.

Hinsichtlich der Komponente b) sind in einer bevorzugten Ausführungsform 1 Gew.-% bis 5 Gew.-% Alkalisilikat mit einem Modul im Bereich von 1,8 bis 2,5 enthalten.

Hinsichtlich der Komponente c) sind in einer bevorzugten Ausführungsform 0,05 Gew.-% bis 1 Gew.-% Phosphonsäure und/oder Alkaliphosphonat enthalten. Unter Phosphonsäuren werden dabei auch gegebenenfalls substituierte Alkylphosphonsäuren verstanden, die auch mehrere Phosphonsäuregruppierungen aufweisen könne (sogenannte Polyphosphonsäuren). Bevorzugt werden sie ausgewählt aus den Hydroxy- und/oder Aminoalkylphosphonsäuren und/oder deren Alkalisalzen, wie zum Beispiel Dimethylaminomethandiphosphonsäure, 3-Aminopropan-1-hydroxy-1,1-diphosphonsäure, 1-Amino-1-phenyl-methandiphosphonsäure, 1-Hydroxyethan-1,1-diphosphonsäure, Amino-tris(methylenphosphonsäure), N,N,N',N'-Ethylendiamin-tetrakis(methylenphos-phonsäure) und acylierte Derivate der phosphorigen Säure, die auch in beliebigen Mischungen eingesetzt werden können.

Hinsichtlich der Komponente d) sind in einer bevorzugten Ausführungsform 15 Gew.-% bis 35 Gew.-% Alkaliphosphat, insbesondere Trinatriumpolyphosphat, enthalten. Alkaliphosphat ist dabei die summarische Bezeichnung für die Alkalimetall- (insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei. Natriumdihydrogenphosphat, NaH₂PO₄, existiert als Dihydrat (Dichte 1,91 gern⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Madrellsches Salz übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° (Zersetzung unter Bildung von (KPO₃)ₓ, Kaliumpolyphosphat) und ist leicht löslich in Wasser. Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gern⁻³, Wasserverlust bei 95°), 7 Mol. (Dichte 1,68 gern⁻³, Schmelzpunkt 48° unter Verlust von 5 H₂O) und 12 Mol. Wasser (Dichte 1,52 gern⁻³, Schmelzpunkt 35° unter Verlust von 5 H₂O), wird bei 100° wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist. Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gern⁻³, hat einen Schmelzpunkt von 1340° und ist in Wasser mit alkalischer Reaktion leicht löslich. Es entsteht z.B. beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt. Tetranatriumdiphosphat (Natriumpyrophosphat), Na₄P₂O₇, existiert in wasserfreier Form (Dichte 2,534 gern⁻³, Schmelzpunkt 988°, auch 880° angegeben) und als Decahydrat (Dichte 1,815-1,836 gern⁻³, Schmelzpunkt 94° unter Wasserverlust). Bei Substanzen sind farblose, in Wasser mit alkalischer Reaktion lösliche Kristalle. Na₄P₂O₇ entsteht beim Erhitzen von Dinatriumphosphat auf >200° oder indem man Phosphorsäure mit Soda im stöchiometrischem Verhältnis umsetzt und die Lösung durch Versprühen entwässert. Das Decahydrat komplexiert Schwermetall-Salze und Härtebitdner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³ dar, das in Wasser löslich ist, wobei der pH-Wert der 1%igen Lösung bei 25° 10,4 beträgt. Durch Kondensation des NaH₂PO₄ bzw. des KH₂PO₄ entstehen höhermolekulare Natrium- und Kaliumphosphate, bei denen man cyclische Vertreter, die Natrium- bzw. Kaliummetaphosphate und kettenförmige Typen, die Natrium- bzw. Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Madrellsches Salz. Alle höheren Natrium- und Kaliumphosphate werden gemeinsam als kondensierte Phosphate bezeichnet. Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.-%-igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind einsetzbar.

Hinsichtlich der Komponente e) sind in einer bevorzugten Ausführungsform der Mittel 1,5 Gew.-% bis 5 Gew.-% polymeres Polycarboxylat, insbesondere ausgewählt aus den Polymerisations- beziehungsweise Copolymerisationsprodukten von Acrylsäure, Methacrylsäure und/oder Maleinsäure enthalten. Unter diesen sind die Homopolymere der Acrylsäure und unter diesen wiederum solche mit einer mittleren Molmasse im Bereich von 5 000 D bis 15 000 D (PA-Standard) besonders bevorzugt.

Als in den Mitteln verwendbare Enzyme kommen außer der obengenannten Oxidase solche aus der Klasse der Proteasen, Lipasen, Cutinasen, Amylasen, Pullulanasen, Mannanasen, Cellulasen, Hemicellulasen, Xylanasen und Peroxidasen sowie deren Gemische in Frage, beispielsweise Proteasen wie BLAP®, Optimase®, Opticlean®, Maxacal®, Maxapem®, Alcalase®, Esperase®, Savinase®, Durazym® und/oder Purafect® OxP, Amylasen wie Termamyl®, Amylase-LT®, Maxamyl®, Duramyl® und/oder Purafect® OxAm, Lipasen wie Lipolase®, Lipomax®, Lumafast® und/oder Lipozym®, Cellulasen wie Celluzyme® und/oder Carezyme®. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind in Wasch-, Reinigungs- und Desinfektionsmitteln vorzugsweise in Mengen bis zu 10 Gew.-%, insbesondere von 0,2 Gew.-% bis 2 Gew.-%, enthalten, wobei besonders bevorzugt gegen oxidativen Abbau stabilisierte Enzyme eingesetzt werden.

In einer bevorzugten Ausführungsform enthält das Mittel 5 Gew.-% bis 50 Gew.-%, insbesondere 8-30 Gew.-% anionisches und/oder nichtionisches Tensid, bis zu 60 Gew.-%, insbesondere 5-40 Gew.-% Buildersubstanz und 0,2 Gew.-% bis 2 Gew.-% Enzym, ausgewählt aus den Proteasen, Lipasen, Cutinasen, Amylasen, Pullulanasen, Mannanasen, Cellulasen, Oxidasen und Peroxidasen sowie deren Gemischen.

Zu den in den Wasch- und Reinigungsmitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den Mitteln vorzugsweise in Mengen nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den Mitteln vorzugsweise nicht über 20 Gew.-%, insbesondere von 1,2 Gew.-% bis 17 Gew.-%, enthalten.

Schmutzablösevermögende Polymere, die oft als "Soil Release"-Wirkstoffe oder wegen ihres Vermögens, die behandelte Oberfläche, zum Beispiel der Faser, schmutzabstoßend auszurüsten, als "Soil Repellents" bezeichnet werden, sind beispielsweise nichtionische oder kationische Cellulosederivate. Zu den insbesondere polyesteraktiven schmutzablösevermögenden Polymeren gehören Copolyester aus Dicarbonsäuren, beispielsweise Adipinsäure, Phthalsäure oder Terephthalsäure, Diolen, beispielsweise Ethylenglykol oder Propylenglykol, und Polydiolen, beispielsweise Polyethylenglykol oder Polypropylenglykol. Zu den bevorzugt eingesetzten schmutzablösevermögenden Polyestern gehören solche Verbindungen, die formal durch Veresterung zweier Monomerteile zugänglich sind, wobei das erste Monomer eine Dicarbonsäure HOOC-Ph-COOH und das zweite Monomer ein Diol HO-(CHR¹¹-)ₐOH, das auch als polymeres Diol H-(O-(CHR¹¹-)ₐ)_{b}OH vorliegen kann, ist. Darin bedeutet Ph einen o-, m- oder p-Phenylenrest, der 1 bis 4 Substituenten, ausgewählt aus Alkylresten mit 1 bis 22 C-Atomen, Sulfonsäuregruppen, Carboxylgruppen und deren Mischungen, tragen kann, R¹¹ Wasserstoff, einen Alkylrest mit 1 bis 22 C-Atomen und deren Mischungen, a eine Zahl von 2 bis 6 und b eine Zahl von 1 bis 300. Vorzugsweise liegen in den aus diesen erhältlichen Polyestern sowohl Monomerdioleinheiten -O-(CHR¹¹-)ₐO- als auch Polymerdioleinheiten -(O-(CHR¹¹-)ₐ)_{b}O- vor. Das molare Verhältnis von Monomerdioleinheiten zu Polymerdioleinheiten beträgt vorzugsweise 100:1 bis 1:100, insbesondere 10:1 bis 1:10. In den Polymerdioleinheiten liegt der Polymerisationsgrad b vorzugsweise im Bereich von 4 bis 200, insbesondere von 12 bis 140. Das Molekulargewicht beziehungsweise das mittlere Molekulargewicht oder das Maximum der Molekulargewichtsverteilung bevorzugter schmutzablösevermögender Polyester liegt im Bereich von 250 bis 100 000, insbesondere von 500 bis 50 000. Die dem Rest Ph zugrundeliegende Säure wird vorzugsweise aus Terephthalsäure, Isophthalsäure, Phthalsäure, Trimellithsäure, Mellithsäure, den Isomeren der Sulfophthalsäure, Sulfoisophthalsäure und Sulfoterephthalsäure sowie deren Gemischen ausgewählt. Sofern deren Säuregruppen nicht Teil der Esterbindungen im Polymer sind, liegen sie vorzugsweise in Salzform, insbesondere als Alkali- oder Ammoniumsalz vor. Unter diesen sind die Natrium- und Kaliumsalze besonders bevorzugt. Gewünschtenfalls können statt des Monomers HOOC-Ph-COOH geringe Anteile, insbesondere nicht mehr als 10 Mol-% bezogen auf den Anteil an Ph mit der oben gegebenen Bedeutung, anderer Säuren, die mindestens zwei Carboxylgruppen aufweisen, im schmutzablösevermögenden Polyester enthalten sein. Zu diesen gehören beispielsweise Alkylen- und Alkenylendicarbonsäuren wie Malonsäure, Bernsteinsäure, Fumarsäure, Maleinsäure, Glutarsäure, Adipinsäure, Pimelinsäure, Korksäure, Azelainsäure und Sebacinsäure. Zu den bevorzugten Diolen HO-(CHR¹¹-)ₐOH gehören solche, in denen R¹¹ Wasserstoff und a eine Zahl von 2 bis 6 ist, und solche, in denen a den Wert 2 aufweist und R¹¹ unter Wasserstoff und den Alkylresten mit 1 bis 10, insbesondere 1 bis 3 C-Atomen ausgewählt wird. Unter den letztgenannten Diolen sind solche der Formel HO-CH₂-CHR¹¹-OH, in der R¹¹ die obengenannte Bedeutung besitzt, besonders bevorzugt. Beispiele für Diolkomponenten sind Ethylenglykol, 1,2-Propylenglykol, 1,3-Propylenglykol, 1,4-Butandiol, 1,5-Pentandiol, 1,6-Hexandiol, 1,8-Octandiol, 1,2-Decandiol, 1,2-Dodecandiol und Neopentylglykol. Besonders bevorzugt unter den polymeren Diolen ist Polyethylenglykol mit einer mittleren Molmasse im Bereich von 1000 bis 6000. Gewünschtenfalls können diese Polyester auch endgruppenverschlossen sein, wobei als Endgruppen Alkylgruppen mit 1 bis 22 C-Atomen und Ester von Monocarbonsäuren in Frage kommen. Den über Esterbindungen gebundenen Endgruppen können Alkyl-, Alkenyl- und Arylmonocarbonsäuren mit 5 bis 32 C-Atomen, insbesondere 5 bis 18 C-Atomen, zugrunde liegen. Zu diesen gehören Valeriansäure, Capronsäure, Önanthsäure, Caprylsäure, Pelargonsäure, Caprinsäure, Undecansäure, Undecensäure, Laurinsäure, Lauroleinsäure, Tridecansäure, Myristinsäure, Myristoleinsäure, Pentadecansäure, Palmitinsäure, Stearinsäure, Petroselinsäure, Petroselaidinsäure, Ölsäure, Linolsäure, Linolaidinsäure, Linolensäure, Eläostearinsäure, Arachinsäure, Gadoleinsäure, Arachidonsäure, Behensäure, Erucasäure, Brassidinsäure, Clupanodonsäure, Lignocerinsäure, Cerotinsäure, Melissinsäure, Benzoesäure, die 1 bis 5 Substituenten mit insgesamt bis zu 25 C-Atomen, insbesondere 1 bis 12 C-Atomen tragen kann, beispielsweise tert.-Butylbenzoesäure. Den Endgruppen können auch Hydroxymonocarbonsäuren mit 5 bis 22 C-Atomen zugrunde liegen, zu denen beispielsweise Hydroxyvaleriansäure, Hydroxycapronsäure, Ricinolsäure, deren Hydrierungsprodukt Hydroxystearinsäure sowie o-, m- und p-Hydroxybenzoesäure gehören. Die Hydroxymonocarbonsäuren können ihrerseits über ihre Hydroxylgruppe und ihre Carboxylgruppe miteinander verbunden sein und damit mehrfach in einer Endgruppe vorliegen. Vorzugsweise liegt die Anzahl der Hydroxymonocarbonsäureeinheiten pro Endgruppe, das heißt ihr Oligomerisierungsgrad, im Bereich von 1 bis 50, insbesondere von 1 bis 10. In einer bevorzugten Ausgestaltung der Erfindung werden Polymere aus Ethylenterephthalat und Polyethylenoxid-terephthalat, in denen die Polyethylenglykol-Einheiten Molgewichte von 750 bis 5000 aufweisen und das Molverhältnis von Ethylenterephthalat zu Polyethylenoxid-terephthalat 50:50 bis 90:10 beträgt, allein oder in Kombination mit Cellulosederivaten verwendet.

Zu den für den Einsatz in Mitteln für die Wäsche von Textilien in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol und gegebenenfalls weiteren Monomeren.

Die Mittel zum Einsatz in der Textilwäsche können Knitterschutzmittel enthalten, da textile Flächengebilde, insbesondere aus Reyon, Wolle, Baumwolle und deren Mischungen, zum Knittern neigen können, weil die Einzelfasern gegen Durchbiegen, Knicken, Pressen und Quetschen quer zur Faserrichtung empfindlich sind. Hierzu zählen beispielsweise synthetische Produkte auf der Basis von Fettsäuren, Fettsäureestern, Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Vergrauungsinhibitoren haben die Aufgabe, den von der harten Oberfläche und insbesondere von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Die Mittel können optische Aufheller, unter diesen insbesondere Derivate der Diaminostilbendisulfonsäure beziehungsweise deren Alkalimetallsalze, enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Insbesondere beim Einsatz in maschinellen Wasch- beziehungsweise Reinigungsverfahren kann es von Vorteil sein, den Mitteln übliche Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt.

In den Mitteln können außerdem Wirkstoffe zur Vermeidung des Anlaufens von Gegenständen aus Silber, sogenannte Silberkorrosionsinhibitoren, eingesetzt werden. Bevorzugte Silberkorrosionsschutzmittel sind organische Disulfide, zweiwertige Phenole, dreiwertige Phenole, gegebenenfalls alkyl- oder aminoalkylsubstituierte Triazole wie Benzotriazol sowie Cobalt-, Mangan-, Titan-, Zirkonium-, Hafnium-, Vanadium- oder Cersalze und/oder -komplexe, in denen die genannten Metalle in einer der Oxidationsstufen II, III, IV, V oder VI vorliegen.

Die genannten Acylhydrazone können in Form von Pulvern oder als Granulate, die auch gegebenenfalls umhüllt und/oder gefärbt sein können und übliche Trägermaterialien und/oder Granulationshilfsmittel enthalten können, vorliegen. Im Fall ihres Einsatzes als Granulate können diese gewünschtenfalls auch weitere Aktivstoffe, insbesondere Bleichaktivator, enthalten.

Die Herstellung fester Mittel bietet keine Schwierigkeiten und kann in im Prinzip bekannter Weise, zum Beispiel durch Sprühtrocknen oder Granulation, erfolgen, wobei empfindliche Inhaltsstoffe wie beispielsweise Persauerstoffverbindung, Enzym oder Bleichaktivator gegebenenfalls später zugesetzt werden. Zur Herstellung der Mittel mit erhöhtem Schüttgewicht, insbesondere im Bereich von 650 g/l bis 950 g/l, ist ein einen Extrusionsschritt aufweisendes Verfahren bevorzugt. Wasch-, Reinigungs- oder Desinfektionsmittel in Form wässriger oder sonstige übliche Lösungsmittel enthaltender Lösungen werden besonders vorteilhaft durch einfaches Mischen der Inhaltsstoffe, die in Substanz oder als Lösung in einen automatischen Mischer gegeben werden können, hergestellt. In einer bevorzugten Ausführung von Mitteln für die insbesondere maschinelle Reinigung von Geschirr sind diese tablettenförmig.

### Beispiele

### Beispiel 1: Herstellung von 1-(2-(2-(2-hydroxybenzyliden)-hydrazinyl)-2-oxoethyl)-1-methyl-piperidinium-bromid,

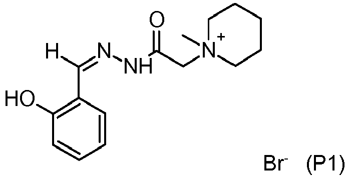

10 ml N-Methylpiperidin wurde in 10 ml Ethylacetat gelöst und auf 2 °C gekühlt. 3,34 g (20 mmol) Bromessigsäureethylester wurden zugetropft; ein Niederschlag fiel aus. Weitere 45 ml Ethylacetat wurden zugegeben und die Reaktionslösung wurde für 16 Stunden gerührt. Anschließend wurde der Feststoff abfiltriert und aus Ethanol/Ethylacetat (1:1, 30 ml) umkristallisiert. Es wurden 4 g (15,4 mmol, 77%) 1-(2-Ethoxy-2-oxoethyl)-1-methylpiperidiniumbromid erhalten.

4 g (15,4 mmol) 1-(2-Ethoxy-2-oxoethyl)-1-methylpiperidiniumbromid wurden in 17 ml Ethanol gelöst. 1,25 g (25 mmol) Hydrazinmonohydrat wurden langsam zugetropft. Die Reaktionslösung wurde 5 Stunden bei Raumtemperatur gerührt und anschließend für 72 Stunden stehengelassen. Das Lösungsmittel wurde unter vermindertem Druck entfernt. Man erhielt 3,78 g (15 mmol, 99%) 1-(2-Hydrazinyl-2-oxoethyl)-1-methylpiperidiniumbromid.

3,6 g (14 mmol) 1-(2-Hydrazinyl-2-oxoethyl)-1-methylpiperidiniumbromid wurden in 20 ml Methanol gelöst. Bei Raumtemperatur wurden unter Rühren 7,5 g Salicylaldehyd (13 mmol) zugetropft, wobei sich die Lösung gelb verfärbte. Die Reaktionslösung wurde für 4 Stunden unter Rühren zum Sieden unter Rückfluss erhitzt und anschließend 72 h bei Raumtemperatur gerührt. Die Reaktionslösung wurde filtriert und der Rückstand getrocknet. Das Filtrat wurde unter vermindertem Druck konzentriert. Die vereinten Feststoffe wurden aus Methanol umkristallisiert. Es wurden 3,15 g (8,84 mmol, 68%) P1 erhalten.

Schmelzpunkt: 171-175 °C.
¹H-NMR (400 MHz, DMSO-de): δ = 12.4 (s, 1Hₐ), 11.9 (s, 1H_{b}), 10.8 (s, 1Hₐ), 10.1 (s, 1H_{b}), 8.6 (s, 1Hₐ), 8.4 (s, 1H_{b}), 7.8 (*m*, 1Hₐ), 7.6 (*m*, 1Hₐ), 7.4-7.2 (*m*, 1Hₐ, 1 H_{b}), 7.0-6.8 (*m*, 1 Hₐ, 3 H_{b}), 4.8 (s, 2H_{b}), 4.4 (s, 2Hₐ), 3.8-3.4 (*m*, 4Hₐ, 4H_{b}), 3.4 (s, 3Hₐ), 3.3 (s, 3H_{b}), 2.0-1.8 (*m*, 4Hₐ, 4H_{b}), 1.9-1.7 (*m*, 2Hₐ, 2H_{b}) ppm.

### Beispiel 2: Waschversuche

Für die Messung der Primärwaschleistung wurden textile Substrate aus den in in der nachfolgenden Tabelle angegebenen Materialien, die mit den in der nachfolgenden Tabelle ebenfalls angegebenen standardisierten Anschmutzungen versehen worden waren, bei 30 °C mit einer Waschlauge enthaltend 3,8 g/l eines teilchenförmigen Waschmittels mit 11,4 Gew.-% Natriumpercarbonat und 3,4 Gew.-% TAED (V1) oder mit einer ansonsten wie V1 zusammengesetzten, aber zusätzlich 0,003 g/l des in Beispiel 1 hergestellten 1-(2-(2-(2-hydroxybenzyliden)-hydrazinyl)-2-oxoethyl)-1-methyl-piperidinium-bromids P1 enthaltenden Waschlauge (M1) gewaschen. Die behandelten Stoffsubstrate wurden anschließend getrocknet und zur Bestimmung des Y-Wertes (Helligkeitswert) farbvermessen. In der nachfolgenden Tabelle ist der Unterschied der Helligkeitswerte der Meßstücke vor und nach der Wäsche (Mittelwert von 6fach-Bestimmungen) angegeben.

**Tabelle 1: Waschleistung**

| Anschmutzung | Textil aus | ΔY bei M1 | ΔY bei V1 |
|---|---|---|---|
| Ruß/Mineralöl | Baumwolle | 49,3 | 45,8 |
| Olivenöl/Ruß | Baumwolle | 50,2 | 47,1 |
| Tomatensauce | Polyester | 69,8 | 68,9 |

## Patentansprüche

1. Verwendung eines Acylhydrazons der allgemeinen Formel I, 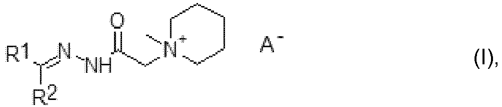 in der der Rest R¹ für einen Rest 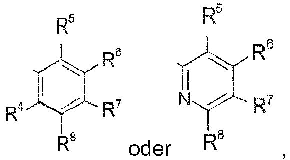
R²=R⁴=R⁶=R⁷=R⁸ für H , R₅ für OH, und
A⁻ für ein ladungsausgleichendes Anion
steht, in Kombination mit einem peroxidischen Bleichmittel zur Verbesserung der Schmutzentfernungsleistung von Wasch- oder Reinigungsmitteln bei deren Anwendung.

2. Verwendung eines Acylhydrazons der allgemeinen Formel I, 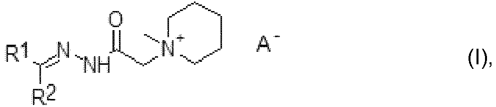 in der der Rest R¹ für einen Rest 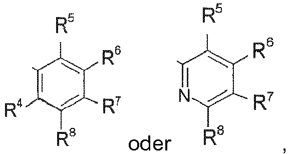
R² = R⁴ = R⁶ = R⁷ = R⁸ für H, R5 für OH, und
A⁻ für ein ladungsausgleichendes Anion
steht, zur Verbesserung der Bleichleistung von peroxidischem Bleichmittel in Wasch- oder Reinigungsmitteln bei deren Anwendung.

3. Verfahren zum Entfernen von Anschmutzungen von textilen oder harten Oberflächen durch In-Kontakt-Bringen der verschmutzten Oberfläche mit einer wässrigen Zubereitung, die Acylhydrazon der allgemeinen Formel I, 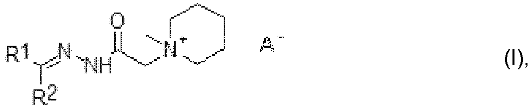 in der der Rest R¹ für einen Rest 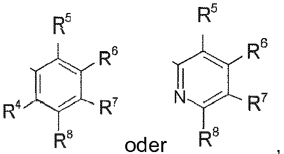
R² = R⁴ = R⁶ = R⁷ = R⁸ für H, R5 für OH, und
A⁻ für ein ladungsausgleichendes Anion steht, und peroxidisches Bleichmittel enthält.

4. Wasch- oder Reinigungsmittel, enthaltend ein Acylhydrazon der allgemeinen Formel I, 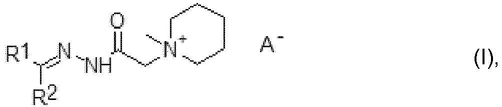 in der in der der Rest R¹ für einen Rest 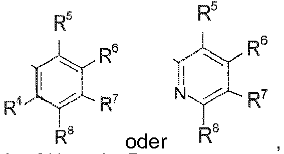
R² = R⁴ = R⁶ = R⁷ = R⁸ für H, R5 für OH, und
A⁻ für ein ladungsausgleichendes Anion
steht, und peroxidisches Bleichmittel.

5. Verwendung nach Anspruch 1 oder 2, Verfahren nach Anspruch 3, oder Mittel nach Anspruch 4, **dadurch gekennzeichnet, dass** das Anion A⁻ in den Acylhydrazonen der allgemeinen Formel (I) aus der Gruppe umfassend Carboxylat wie Acetat, Lactat, Citrat, Tartrat oder Succinat, Perchlorat, Tetrafluoroborat, Hexafluorophosphat, Alkylsulfonat, Alkylsulfat, Hydrogensulfat, Sulfat, Dihydrogenphosphat, Hydrogenphosphat, Phosphat, Isocyanat, Rhodanid, Nitrat, Fluorid, Chlorid, Bromid, Hydrogencarbonat und Carbonat sowie deren Mischungen ausgewählt wird, wobei bei mehrwertigen Anionen der Ladungsausgleich durch entsprechend mehrere kationische Acylhydrazone oder gegebenenfalls durch die Anwesenheit zusätzlicher Kationen wie Natrium- oder Ammoniumionen erreicht wird.

## Claims

1. The use of an acylhydrazone of the general formula I, 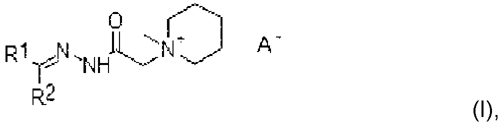 in which the functional group R¹ represents a functional group 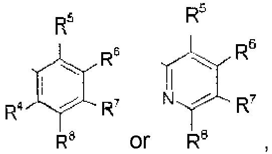
R² = R⁴ = R⁶ = R⁷ = R⁸ represents H, R⁵ represents OH, and
A⁻ represents a charge-balancing anion,
in combination with a peroxide bleaching agent, for improving the dirt-removal performance of washing or cleaning agents when said agents are used.

2. The use of an acylhydrazone of the general formula I, 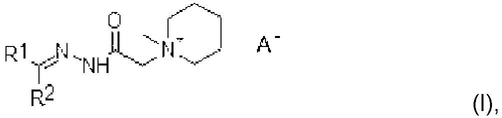 in which the functional group R¹ represents a functional group 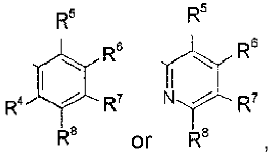
R² = R⁴ = R⁶ = R⁷ = R⁸ represents H, R⁵ represents OH, and
A⁻ represents a charge-balancing anion,
for improving the bleaching performance of a peroxide bleaching agent in washing or cleaning agents when said agents are used.

3. A method for removing stains from textile or hard surfaces by bringing the soiled surface into contact with an aqueous preparation which contains an acylhydrazone of the general formula I, 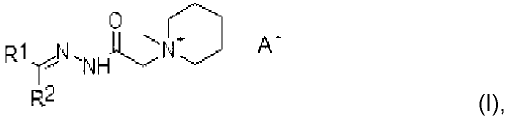 in which the functional group R¹ represents a functional group 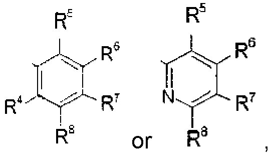
R² = R⁴ = R⁶ = R⁷ = R⁸ represents H, R⁵ represents OH, and
A⁻ represents a charge-balancing anion,
and a peroxide bleaching agent.

4. A washing or cleaning agent containing an acylhydrazone of the general formula I, 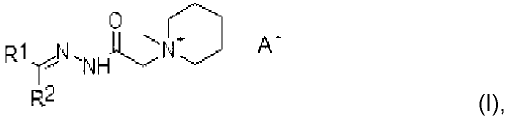 in which the functional group R¹ represents a functional group 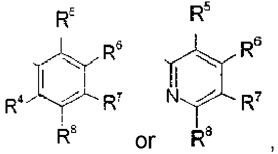
R² = R⁴ = R⁶ = R⁷ = R⁸ represents H, R⁵ represents OH, and
A⁻ represents a charge-balancing anion,
and a peroxide bleaching agent.

5. The use according to claim 1 or 2, the method according to claim 3 or the agent according to claim 4, **characterized in that** the anion A⁻ in the acylhydrazones of the general formula (I) is selected from the group comprising carboxylate, such as acetate, lactate, citrate, tartrate or succinate, perchlorate, tetrafluoroborate, hexafluorophosphate, alkyl sulfonate, alkyl sulfate, hydrogen sulfate, sulfate, dihydrogen phosphate, hydrogen phosphate, phosphate, isocyanate, thiocyanate, nitrate, fluoride, chloride, bromide, hydrogen carbonate and carbonate and mixtures thereof, charge balance being achieved, in the case of polyvalent anions, by means of a corresponding number of cationic acylhydrazones or optionally by means of the presence of additional cations such as sodium or ammonium ions.

## Revendications

1. Utilisation d'une acylhydrazone de formule générale I, 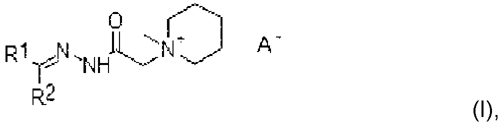 dans laquelle le radical R¹ représente un radical 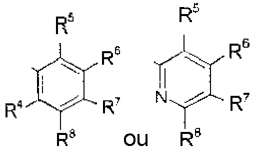
R² = R⁴ = R⁶ = R⁷ = R⁸ représentent H, R⁵ représente OH et
A⁻ représente un anion équilibrant la charge, en combinaison avec un agent de blanchiment peroxydique pour améliorer les performances d'élimination des salissures des détergents ou des compositions de nettoyage lorsqu'ils sont utilisés.

2. Utilisation d'une acylhydrazone de formule générale I, 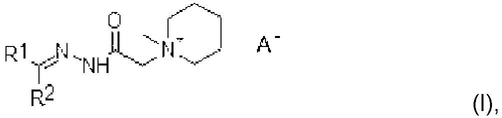 dans laquelle le radical R¹ représente un radical 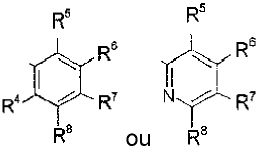
R² = R⁴ = R⁶ = R⁷ = R⁸ représentent H, R⁵ représente OH et
A⁻ représente un anion équilibrant la charge, pour améliorer la performance de blanchiment d'agents de blanchiment peroxydiques dans les détergents ou les compositions de nettoyage lorsqu'ils sont utilisés.

3. Procédé d'élimination de taches sur des surfaces textiles ou dures par mise en contact de la surface souillée avec une préparation aqueuse contenant de l'acylhydrazone de formule générale I, 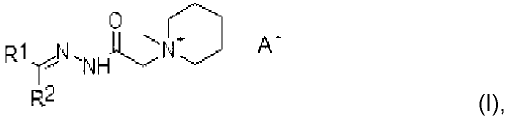 dans laquelle le radical R¹ représente un radical 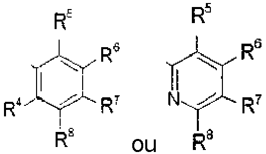
R² = R⁴ = R⁶ = R⁷ = R⁸ représentent H, R⁵ représente OH et
A⁻ représente un anion équilibrant la charge, et un agent de blanchiment au peroxyde.

4. Agent détergent ou agent de nettoyage contenant une acylhydrazone de formule générale I, 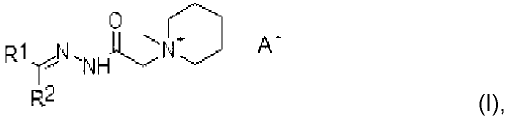 dans laquelle dans laquelle le radical R¹ représente un radical 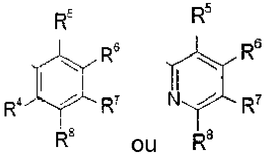
R² = R⁴ = R⁶ = R⁷ = R⁸ représentent H, R⁵ représente OH et
A⁻ représente un anion équilibrant la charge, et un agent de blanchiment au peroxyde.

5. Utilisation selon la revendication 1 ou 2, procédé selon la revendication 3, ou agent selon la revendication 4, caractérisé(e) en ce que l'anion A⁻ dans les acylhydrazones de formule générale (I) est choisi dans le groupe comprenant un carboxylate tel qu'un acétate, un lactate, un citrate, un tartrate ou un succinate, un perchlorate, un tétrafluoroborate, un hexafluorophosphate, un alkylsulfonate, un alkylsulfate, un hydrogénosulfate, un sulfate, un dihydrogénophosphate, un hydrogénophosphate, un phosphate, un isocyanate, un rhodanide, un nitrate, un fluorure, un chlorure, un bromure, un hydrogénocarbonate et un carbonate ainsi que leurs mélanges, dans lequel, dans le cas d'anions polyvalents, l'équilibre des charges est obtenu par plusieurs acylhydrazones cationiques correspondantes ou éventuellement par la présence de cations supplémentaires tels que des ions sodium ou ammonium.
